# EUROPÄISCHE PATENTSCHRIFT

(11) **EP 1 880 670 B1**
(45) Veröffentlichungstag und Bekanntmachung des Hinweises auf die Patenterteilung: **15.12.2010**
(21) Anmeldenummer: 06015187.5
(22) Anmeldetag: 21.07.2006
(51) Int. Cl.: A61B 5/151

(54) **Multilanzetten**
Multi-lancets
Multi-lancettes

(43) Veröffentlichungstag der Anmeldung: 23.01.2008
(73) Patentinhaber: Roche Diagnostics GmbH, 68298 Mannheim (DE); F.Hoffmann-La Roche AG, 4070 Basel (CH)
(72) Erfinder: Hoenes, Joachim, 64673 Zwingenberg (DE); Zimmer, Volker, 67229 Laumersheim (DE); Voelkel, Dirk, 69469 Weinheim (DE); Haar, Hans-Peter, 69168 Wiesloch (DE); Quarder, Ortrud, 69115 Heidelberg (DE); Kuhr, Hans-Jürgen, 68219 Mannheim (DE); Harttig, Herbert, 67434 Neustadt (DE)

(56) Entgegenhaltungen:
- GB-A- 1 211 432
- US-A- 6 132 449
- US-B1- 6 589 202

## Beschreibung

### Technisches Gebiet

Die Erfindung liegt auf dem Gebiet der Stechhilfen zur diagnostischen Ermittelung von Blutparametern.

### Stand der Technik

Die Gewinnung und Analyse von Körperflüssigkeiten findet auf vielen Gebieten der medizinischen Diagnostik statt. Deshalb ist es wünschenswert auch Routinetests außerhalb des Laboratoriums schnell und reproduzierbar zu ermöglichen. Das Testen kann mit verschiedenen Körperflüssigkeiten durchgeführt werden, wie z. B. Blut und/oder interstitieller Flüssigkeit. Diese Flüssigkeiten können auf verschiedene Charakteristiken hin untersucht werden. Die Ergebnisse dieser Untersuchung sind wichtig, um verlässliche Diagnosen, therapeutische Maßnahmen und Therapieverfolgungen durchführen zu können.

Die Analyse von Körperflüssigkeiten beginnt mit der Gewinnung der Flüssigkeit. Eine Methode zur Gewinnung von Körperflüssigkeit besteht darin, eine minimale Wunde in die Haut des Patienten mit Hilfe einer Nadel, Lanzette oder eines Messers zu erzeugen. Die dabei gewonnene Körperflüssigkeit kann entweder in kleinen Gefäßen gesammelt werden oder direkt in Kontakt mit einem Testelement wie z. B. einem Teststreifen zur Analyse gebracht werden. Die meisten dieser Stechhilfen benötigen ein manuelles Einfügen der Lanzette in die Stechhilfe. Dies ist bei einer sehr häufigen Benutzung der Stechhilfe eine sehr umständliche Handhabung. Eine Magazinierung von Lanzetten kann dieses Problem beheben, wobei hier viele Sicherheitsaspekte zu beachten sind. Es ist beispielsweise zu beachten, dass die Sicherheit des Patienten bei der Benutzung der Stechhilfe gewährleistet ist. Zudem sollte das System nicht zu komplex werden, da es sonst vom Patienten nicht gut handhabbar wäre. Ein weiteres wichtiges Merkmal neben der Magazinierung, ist die Möglichkeit zur Einstellung der Stechtiefe vor dem Stechvorgang. Dies kann Geräteseitig gewährleistet werden, wie dies beispielsweise in der Patentanmeldung WO 2006038044 beschrieben ist. Durch die Geräteseitige Einstellung der Stechtiefe ist ein sehr aufwendiger Mechanismus notwendig, da Antrieb der Lanzette und der Stechtiefenregelungsmechanismus auf einander eingestellt werden müssen. Alternativ könnte die Stechtiefe auch durch die Lanzetten gesteuert werden.
Im Stand der Technik werden zwar Stechhilfen beschrieben, die mehr als eine Lanzette aufweisen, jedoch gibt es hier keine Möglichkeit für den Benutzer vor dem Einstich die Stechtiefe zu beeinflussen. In der US 4,794,926 wird beispielsweise ein Lanzettensystem beschrieben, in dem an einem Grundkörper mehrere Lanzetten bevorratet werden, die einzeln zum Stechvorgang benutzt werden können. Jedoch bietet dieses System ebenfalls keine Möglichkeit zur Wahl der Stechtiefe für den Patienten vor dem Stechvorgang.

Das Dokument GB 1211432 beschreibt hingegen eine Lanzette, die zwei Lanzettenspitzen mit unterschiedlichen Längen aufweist, sodass die Lanzette sowohl für Kinder als auch für Erwachsene benutzt werden kann.

Aus den Nachteilen des Standes der Technik ergibt sich die Aufgabe, eine Stechhilfe zu generieren, die leicht handhabbar ist und kostengünstig herzustellen ist und dabei eine einfache Regulierung der Stechtiefe ermöglicht.

Diese Aufgabe wird durch den Gegenstand der Erfindung, wie er in den unabhängigen Patentansprüchen charakterisiert wird, gelöst. Bevorzugte Ausführungsformen sind Gegenstand der abhängigen Ansprüche.

Gegenstand der Erfindung ist eine Vorrichtung zur Gewinnung von Körperflüssigkeiten, die mindestens eine Lanzette, beinhaltend einen Lanzettenkörper mit mindestens zwei Spitzen unterschiedlicher Länge aufweist. Die Vorrichtung ist **dadurch gekennzeichnet, dass** sie ein Mittel zur Selektion besitzt, mit dessen Hilfe vor dem Einstich nur eine Spitze ausgewählt wird. Dieses Mittel zur Selektion kann verschiedene Funktionsweisen und Formen aufweisen. Das Mittel zur Selektion dient dazu, aus einer Mehrzahl von Lanzettenspitzen, die unterschiedliche Längen aufweisen, eine Spitze auszuwählen und nur diese ausgewählte Spitze zur Benutzung für den Stechvorgang bereit zu stellen. Da die Lanzettenspitzen unterschiedliche Längen aufweisen, kann die Stechtiefe durch die Wahl der zum Stechvorgang zu benutzenden Lanzettenspitze eingestellt werden. Beim Stechvorgang selbst sind die ausgewählte Lanzettenspitze und die weiteren Lanzettenspitzen so angeordnet, dass nur die ausgewählte Lanzettenspitze in den zu stechenden Körper eindringt.

Diese Vorrichtung zur Gewinnung von Körperflüssigkeit hat den Vorteil, dass am Gerät selber keine weitere Regelung der Stechtiefe vorgenommen werden muss, wie dies im Stand der Technik (z.B. WO 20060380449) der Fall ist. Hierdurch braucht auch keine besondere Abstimmung des Antriebs mit der geräteseitigen Stechtiefenregelung erfolgen. Am Geräte selber brauchen keine verstellbaren Teile angebracht und justiert zu werden. Das hat zur Folge, dass kostengünstige Antriebe und Gehäuse verwendet werden können.
In der hier beschriebenen Vorrichtung findet die Auswahl der Stechtiefe also über die Auswahl der Lanzettenspitze statt. Dies bedeutet, dass mehrere Lanzettenspitzen mit unterschiedlichen Längen bereitgestellt werden müssen. Diese unterschiedlich langen Lanzettenspitzen sind an einem Lanzettenkörper angeordnet. Die Art der Anordnung kann dabei alle möglichen Formen und Geometrien beinhalten. So können die Lanzettenspitzen linear angeordnet sein, wobei die Spitzen in die gleiche oder in entgegen gesetzte Richtungen weisen können. Die Lanzettenspitzen können aber auch nicht linear in verschiedene Richtungen weisen, indem sie beispielsweise in unterschiedlichen Winkeln an dem Lanzettenkörper angeordnet sein. Eine bevorzugte Ausführungsform mit verschiedenen Lanzettenspitzen, die in unterschiedlichen Richtungen am Lanzettenkörper angeordnet sind, ist eine sternförmige Anordnung der Spitzen. In einer besonders bevorzugten Ausführungsform sind die Lanzettenspitzen in einer Ebene an dem Lanzettenkörper angebracht.

Nach Auswahl einer bestimmten Lanzettenspitze, kann die einzelne Spitze benutzt werden, in dem der Lanzettenkörper so bewegt wird, dass nur eine Lanzettenspitze bei Bewegung des Lanzettenkörpers in die Richtung des stechenden Körpers weist. Die anderen Lanzettenspitzen weisen in andere Richtungen und werden folglich beim Einstichvorgang nicht mit in den Körper eingestochen. Diese Anordnung von ausgewählten und nicht ausgewählten Spitzen kann durch verschiedene Geometrien des Lanzettenkörpers auf unterschiedliche Weise realisiert werden. Hierzu sind verschiedene Mittel zur Selektion nötig.

Für die verschiedenen Anordnungen der Lanzettenspitzen können verschiedene Mittel zur Selektion der zu benutzenden Spitze bereitgestellt werden. Dieses Mittel zur Selektion kann beispielsweise eine Markierung an der Lanzette selbst oder am Lanzettenkörper beinhalten. Solch eine Markierung kann beispielsweise optisch, mechanisch oder elektrochemisch in der Vorrichtung erkannt werden. Hierfür kann ein Sensor dienen, der es ermöglicht eine optische, mechanische oder elektrochemische Registrierung der Anordnung des Lanzettenkörpers in der Vorrichtung vorzunehmen. Diese Markierung kann beispielsweise eine Einkerbung sein oder aber auch eine Einfärbung eines Teils der Lanzetten bzw. einem möglichen Träger, falls die Lanzette auf einem Träger aufgebracht ist.

Weiterhin kann das Mittel zur Selektion eine Vorrichtung sein, die es ermöglicht den Lanzettenkörper so zu positionieren, dass die ausgewählte Lanzettenspitze zur Benutzung bereitgestellt wird, sodass sie mit einer Antriebseinheit Wechsel wirken kann, um zum Einstich bewegt zu werden. Dies kann beispielsweise die gleiche Vorrichtung sein, die auch zum Weitertakten der Lanzettenkörper benutzt wird. Bei jedem Weitertakten wird der Lanzettenkörper um eine Lanzettenspitze weiter verschoben. Dies kann dem Benutzer über einen Zähler angezeigt werden, sodass für den Benutzer immer ersichtlich ist, welche Lanzettenlänge gerade zum Einstich bereit gestellt ist. Dabei sollte verhindert werden, dass die Vorrichtung zurück getaktet werden kann, damit keine wiederholte Benutzung einer Lanzettenspitze möglich ist. Aber auch jede andere Art der Selektion ist möglich, die es ermöglicht eine Positionierung des Lanzettenkörpers zu gewährleisten, um eine einzelne Lanzettenspitze zur Benutzung auszuwählen und zum Einstich so zur Antriebseinheit zu positionieren, ohne, dass mehr als eine Lanzettenspitze zur Benutzung kommt. Auch bei der sternförmigen Anordnung der Lanzettenspitzen am Lanzettenkörper sowie der Anordnung von verschiedenen Spitzen in unterschiedlichen Winkeln am Lanzettenkörper kann eine Markierung optischer oder mechanischer Natur wie oben beschrieben vorgenommen werden. Weitere Möglichkeiten zur Markierung des Lanzettenkörpers sind eine unterschiedliche Magnetisierung bzw. unterschiedliche Impedanz verschiedener Bereiche des Lanzettenkörpers oder der Lanzettenspitzen. Bei der optischen Erkennung des Lanzettenkörpers bzw. der einzelnen Lanzettenspitzen benötigt die Vorrichtung ein optisches Erkennungsmittel wie beispielsweise einen optischen Sensor. Hingegen wird bei der mechanischen Erkennung der Positionierung des Lanzettekörpers beispielsweise ein Einrastemechanismus benötigt, der es ermöglicht, dass Einkerbungen im Lanzettenkörper zu einer exakten Positionierung des Lanzettenkörpers und damit der Lanzettenspitzen führt. Unabhängig von der Art und Weise der Selektion, der vom Benutzer ausgewählten Lanzettenspitze, ist es wichtig durch die Selektion eine exakte Positionierung des Lanzettenkörpers zu erreichen. Durch Selektion und anschließend exakte Positionierung des Lanzettenkörpers wird gewährleistet, dass die Antriebseinheit den Lanzettenkörper in der Art bewegt, dass nur die ausgewählte Lanzettenspitze in den Körper eindringt.

Wenn in diesem Zusammenhang von Positionieren gesprochen wird, so ist die Positionierung der ausgewählten und selektierten Lanzettenspitze bzw. des Lanzettenkörpers in Bezug auf die Antriebseinheit einerseits und den Einstichort gemeint. Es soll also gewährleistet sein, dass die selektierte Lanzettenspitze von der Antriebseinheit bewegt werden kann, sodass ein Einstich an dem zum Einstich bereitgestellten Körperteil erfolgen kann.

Alternativ zu einer Markierung eines Teils der Lanzette oder des Trägers der Lanzette, kann der Träger bzw. die Lanzette so genau in der Vorrichtung positioniert sein, dass allein durch Rotation und/oder Verschieben des Lanzettenkörpers oder des Trägers die ausgewählte Lanzettenspitze zur Benutzung bereitgestellt werden kann.

Da die verschiedenen Spitzen an einem Lanzettenkörper angebracht sind, kann für die Bereitstellung von nur einer Lanzettenspitze für die Benutzung beispielsweise ein Knickelement verwendet werden, welches eine der verschiedenen Spitzen aus der Lanzettenkörperebene abknickt. Dies ist besonders bevorzugt bei Lanzettenspitzen, die linear zueinander angeordnet sind. Hierbei bildet das Knickelement das Mittel zur Selektion. Das Abknicken ermöglicht es, bei Bewegung des Lanzettenkörpers zum Einstich, nur die abgeknickte Lanzette in Richtung des zu stechenden Körperteils zu bewegen. Durch das Abknicken wird folglich verhindert, dass mehr als eine Lanzettenspitze in den Körper eindringt.

Eine Alternative zum Abknicken der ausgewählten Lanzettenspitze, ist die ausreichende Beabstandung der Lanzettenspitzen zueinander, so dass eine Spitze positioniert und benutzt werden kann, ohne dass weitere Spitzen in den Körper eindringen, obwohl alle Spitzen insbesondere auch in gleicher Richtung an einem Lanzettenkörper angeordnet sind. Selbst bei gleichzeitiger Bewegung sämtlicher Lanzettenspitzen beim Einstichvorgang kann durch die Beabstandung gewährleistet werden, dass nur eine Lanzettenspitze in den Körper eindringt. Hier ist folglich das Mittel zur Selektion aufgrund einer ausreichenden Beabstandung der Lanzettenspitzen realisierbar. Vorteilhafterweise weist der Lanzettenkörper somit mehrere Kopplungselemente auf, die ein Ankoppeln der Lanzette an die Antriebseinheit an verschiedenen Stellen des Lanzettenkörpers ermöglichen. In Abhängigkeit des ausgewählten Kopplungselementes erfolgt entsprechend eine Selektion der Lanzettenspitze, die für einen Stechvorgang verwendet werden soll. An der beschriebenen Ausführungsform sind folglich die Kopplungselemente Bestandteil des Mittels zur Selektion.

Wenn die Lanzettenspitze vom Lanzettenkörper abgeknickt werden soll, so sollte die Lanzette besondere Materialeigenschaften aufweisen. So kann die Lanzette beispielsweise eine Flachlanzette sein und/oder einen Knickbereich aufweisen. In einer bevorzugten Ausführungsform ist die Lanzette eine Flachlanzette und besitzt einen Knickbereich. In einer besonders bevorzugten Ausführungsform ist der Lanzettenkörper ebenfalls flach ausgestaltet. Der Knickbereich der Lanzette, der auch außerhalb des Spitzenbereiches liegen kann, weist mindestens eine Struktur mit veränderter Steifigkeit auf. Diese mindestens eine Struktur mit veränderter Steifigkeit wird im Folgenden als Prägung bezeichnet. Die Prägung kann durch z. B. Ätzen, Stanzen oder Hämmern oder sonstige Metall bearbeitende Maßnahmen in oder auf die Lanzette gearbeitet werden. Die Steifigkeit kann also vorzugsweise durch die Variation der Geometrie der Lanzette oder durch die Variation der Materialmenge zur Produktion der Lanzette eingestellt werden. Eine bevorzugte Ausführungsform beinhaltet mehr als eine Prägung in dem Knickbereich der Lanzette. Eine besonders bevorzugte Ausführungsform dieser Prägung ist eine Dreifachprägung im Knickbereich der Lanzette, die sich über mindestens einen Teil der Längsausdehnung der Lanzette erstreckt. Eine Prägung erstreckt sich dabei von dem distalen Ende der Lanzette in axialer Richtung in Richtung zum proximalen Ende der Lanzette. Die Länge der Prägung ist variabel. Diese Prägung kann von zwei Seiten in die Flachlanzette eingebracht sein. Diese unterschiedliche Richtung der Prägung bewirkt, dass die Lanzettenspitze in die entgegen gesetzte Richtung zum Lanzettenkörper abknickt. Hierdurch werden die abgeknickten Flächen in einem Winkel von vorzugsweise bis zu 100 ° aus der Lanzettenkörperebene gehoben. Dadurch wird die Lanzettenspitze aus der Ebene des Lanzettenkörpers herausbewegt.

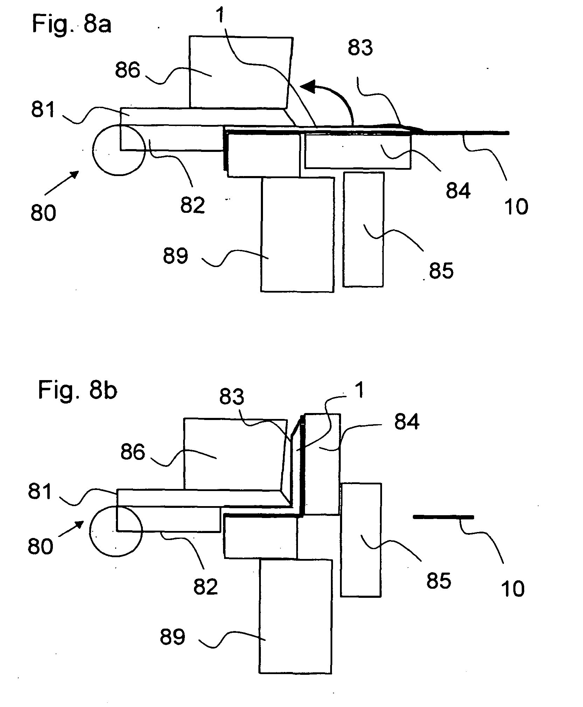

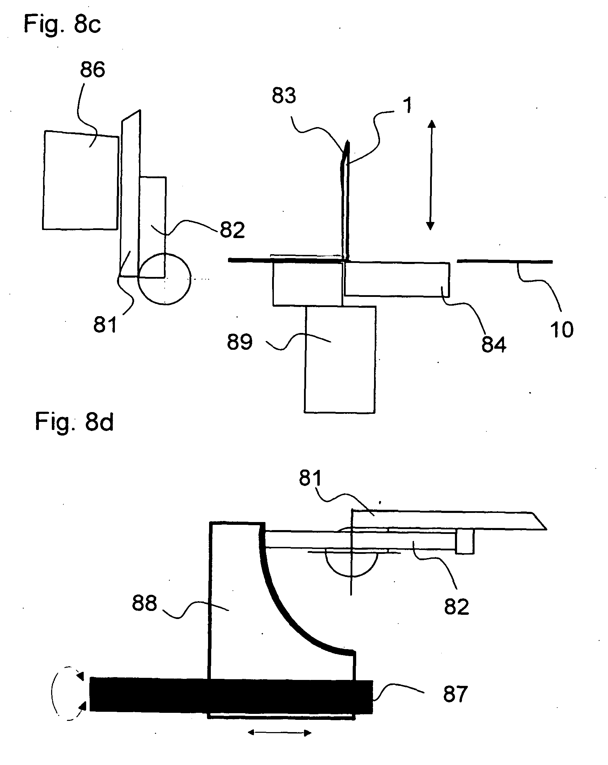

Die Übertragung der Kraft auf die Lanzette kann durch ein Knickelement, z.B. einem Stößel stattfinden, der auf die Lanzette gedrückt wird. Bei einer speziellen Ausführungsform mit mehr als einer Prägung, besteht die Möglichkeit die Kraft auf die Lanzette zu übertragen indem beispielsweise der Lanzettenkörper mit der Lanzettenspitze über den Stößel geleitet wird bzw. der Stößel gegen die Lanzettenspitze drückt. Hierbei wirkt eine ausreichend große Kraft (Schwellenkraft) auf die Lanzette, um die Lanzettenspitze aus der Lanzettenkörperebene herauszubewegen. Ist der Lanzettenkörper ein Trägerband oder ist der Lanzettenkörper auf einem Trägerband fixiert, so kann es nötig sein, die Lanzettenspitze nach dem Einstich wieder in die Trägerbandebene zurück zu knicken, um eine Remagazinierung zu ermöglichen. Hierzu kann ein Rückstellelement dienen, das nach dem Einstich die Lanzettenspitze wieder in ihre ursprüngliche Position vor dem Abknicken überführt, wie dies in den Figuren 8 a-d dargestellt ist. Weiterhin kann die Remagazinierung aufgrund der Materialeigenschaft der Lanzette geschehen. So kann die Lanzette aus einem Formgedächtnismaterial, wie beispielsweise Nitinol gefertigt sein, das sich im kühlen Zustand (wie beispielsweise Raumtemperatur) biegen lässt und die gebogene Form solange erhalten bleibt, bis es erwärmt wird. Diese Rückbiegetemperatur liegt je nach Legierung zwischen 34-60°C.

Eine bevorzugte Ausführungsform für die Anordnung der verschieden langen Lanzettenspitzen ist ein Lanzettenrad. Hierbei werden kreisförmig Lanzetten in einer Ebene angeordnet, die unterschiedliche lang Lanzettenspitzen aufweisen, oder deren Lanzettenspitzen an unterschiedlichen Stellen geknickt werden, um die Länger der Lanzettenspitze zu variieren. Dabei kann die Lanzettenspitze auch in Form einer Schneide ausgebildet sein.

Das Material der Lanzette ist bevorzugter weise Metall, besonders bevorzugt ist Stahl. Die Lanzette kann aber auch aus anderen Materialien und Materialkombinationen bestehen. Außerdem sollte das Material der Gestalt sein, dass es an dem distalen Ende der Lanzettenspitze zu einer scharfen Spitze gearbeitet werden kann, da sonst beim Stich zu viel Schmerz generiert wird. Die Herstellung von Lanzetten im Allgemeinen ist im Stand der Technik hinreichend bekannt, wie beispielsweise in DE 19604156 oder EP 0565970. Wird die Lanzette vor dem Stechvorgang geknickt, so sollte das Material der Lanzette der Gestalt sein, dass die Lanzette bei Krafteinwirkung knickbar ist und dabei genügend Steifigkeit besitzt, um bei Benutzung in die Haut einzudringen ohne ihre Form zu verändern. In einer bevorzugten Ausführungsform ist der Lanzettenkörper zusammen mit den Lanzettenspitzen in einem Stück gearbeitet. Dies kann beispielsweise durch Ätzen oder Stanzen der Lanzette aus Stahl erzeugt werden.

Zur Aktuierung der Lanzette können verschiedene Methoden verwendet werden. Die Wahl der Methode hängt vor allem von der Geometrie der Vorrichtung ab. So kann die Vorrichtung ausschließlich eine Lanzette antreiben, oder die Vorrichtung kann in einem System mit weiteren Elementen, wie Testelementen, Detektoren, Auswerteeinheiten und Anzeigeeinheiten kombiniert werden. In einer bevorzugten Ausführungsform werden mehrere Lanzettenkörper mit mehreren Testelementen auf oder an einem Trägerband angeordnet. Das Trägerband kann hier als Lanzettenkörper fungieren. Die Lanzette kann beispielsweise an ihrem proximalen Ende auf dem Trägerband so fixiert sein, dass ein Teil der Lanzette in Relation zum oder mit dem Trägerband bewegt werden kann, während das proximale Ende an mindestens einem Punkt mit dem Trägerband verbunden ist. Eine weitere bevorzugte Befestigung der Lanzette, ist die Fixierung des Lanzettenkörpers an dem Trägerband, wobei sich der Spitzenbereich vom Trägerband löst. Die gesteuerte Bewegung der Lanzette kann durch Bewegen des Trägerbandes oder durch Ergreifen der Lanzette mit einem Greifelement geschehen, wobei die Lanzettenspitze mit dem Trägerband aus der Ebene des Trägerbandes herausbewegt wird. Diese Bewegung kann mittels eines Antriebselementes durchgeführt werden, das senkrecht zur Trägerbandebene auf die Lanzette Kraft überträgt. Die Kraftübertragung findet durch ein Antriebselement statt, das z.B. ein Stößel sein kann oder ein Greifelement, welches die Lanzette an ihrem Lanzettenkörper greift und bewegt. Hierbei ist in einer bevorzugten Ausführungsform die Einstichtiefe des Blutentnahmegerätes frei wählbar. Zur Regulierung der Einstichtiefe kann zusätzlich zur Länge der Lanzettenspitze die Bewegung der Lanzette durch ein variierbares Anschlagselement definiert werden, gegen das die Lanzette während des Einstichvorgangs anschlägt. In Abhängigkeit von der Position des Anschlagelementes wird auf diese Weise die Länge der Lanzettenspitze, die in den Körper einsticht und somit die Einstichtiefe variiert. Das Anschlagselement kann beispielsweise in das Gehäuse integriert werden. Des Weiteren kann die Lanzette selbst als Anschlagselement dienen, wobei die Stechtiefe durch die Länge der aus dem Lanzettenkörper herausragenden ggf. abgeknickten Spitze definiert wird. Hierbei bildet der Lanzettenkörper eine Barriere für das weitere Eindringen der Lanzette in die Haut.

Zum Antrieb der Lanzette können ballistische oder Kulissen geführte Mechanismen benutzt werden, die aus dem Stand der Technik bekannt sind, wie z.B. in DE 19604156, EP 0565970, US 5318584 oder US 4924879 beschrieben. Eine bevorzugte Ausführungsform für den Antrieb der Lanzette ist die freie Bewegung der Lanzette nach Kraftübertragung durch das Antriebselement, wie beispielsweise dem Stößel. In dieser Ausführungsform wird ein Impuls von dem Antriebselement auf die Lanzette übertragen und die Lanzette bewegt sich ohne weitere Führung durch das Antriebselement in Richtung Gehäuseöffnung. Die Bewegung der Lanzette kann dabei durch zusätzliche Elemente am Gehäuse geführt werden.

Zur hygienischen Verwendung des Systems wird die Lanzette mindestens der Spitzenbereich der Lanzette durch einen Sterilschutz geschützt. Dieser Sterilschutz kann eine Folie sein, die zusätzlich noch ein antibakterielles Mittel, wie beispielsweise Silber, enthält. Bevorzugter weise ist die Lanzette über den ganzen Lanzettenkörper mit dieser Folie als Sterilschutz überdeckt. Die Folie kann sich, bei Verwendung eines Trägerbandes auch über einen Teil des Trägerbandes erstrecken und damit verbunden sein. Dieser Sterilschutz kann aus einer Polymerschicht bestehen, die nach der Herstellung der Lanzette aufgebracht wird. Diese Polymerschicht wird beim Aufwenden der Schwellenkraft auf die Lanzettenspitze von der Lanzettenspitze zerstört oder durchstochen und legt die Lanzettenspitze frei. Alternativ kann der Sterilschutz vor Benutzung der Lanzette entfernt werden. Bevorzugter weise wird der Sterilschutz hierbei im Ganzen entfernt.

In einer bevorzugten Ausführungsform weist die Lanzette mindestens in ihrem Spitzenbereich einen Kanal auf, der dazu dient, Blut aus der Wunde mit der Lanzette zu sammeln. Diese Ausführungsform wird im Folgenden als Microsampler bezeichnet. Das im Microsampler gesammelte Blut kann anschließend auf ein Testelement übertragen werden und durch ein Detektionssystem (z. B. optisch oder elektrochemisch) detektiert und durch ein Auswertesystem ausgewertet werden.

Gegenstand der Erfindung ist weiterhin eine Lanzette zum Einstechen in einen Körperteil.

Erfindungsgemäß weist der Lanzettenkörper mindestens zwei Spitzen auf, die unterschiedliche Längen besitzen. Die Lanzette ist dabei derartig ausgestaltet, dass eine Spitze auswählbar ist, so dass ein Stechvorgang mit definierter Einstichtiefe in Abhängigkeit von der Länge der ausgewählten Spitze erfolgen kann. Erfindungsgemäß sind des Weiteren die nicht ausgewählten Spitzen derartig während eines Stechvorgangs relativ zu der ausgewählten Spitze positionierbar, dass ausschließlich mit der ausgewählten Spitze ein Stechvorgang ausgeführt wird, während die nicht ausgewählte Spitze nicht am Stechvorgang beteiligt ist. Auf diese Weise ist einen Regulierung der Einstichtiefe durch die Wahl/ Selektion einer bestimmten Spitze der Lanzette möglich. Beispielsweise kann eine Selektion einer Lanzettenspitze durch das Abknicken einer Spitze erfolgen, wobei die Lanzette hierfür wie bereits beschrieben einen Knickbereich aufweist. Es ist aber auch möglich ein Kopplungselement einer Lanzette derartig auszugestalten, dass eine Antriebseinheit an unterschiedlichen Positionen der Lanzette Ankoppeln kann oder die Lanzette in unterschiedlichen Positionen an die Antriebseinheit gekoppelt wird, so dass hierdurch eine Spitze zum Ausführen eines Stechvorgangs selektiert wird. Allgemein ist als Kopplungselement der Teil der Lanzette zu verstehen, der ein Ankoppeln zwischen Lanzette und Antriebseinheit einer Vorrichtung ermöglicht. Beispielsweise kann ein Kopplungselement eine Ausnehmung im Lanzettenkörper beinhalten, in die eine Antriebseinheit eingreifen kann. Es sind aber auch andere Ausgestaltungen möglich, wie z.B. Strukturierungen des Lanzettenkörpers, wie sie bereits im Stand der Technik hinreichend bekannt sind und bei Systemen beschrieben werden, die ein Ankoppeln einer zum Auswechseln vorgesehenen Lanzette an ein Blutentnahmesystem erlauben. Darüber hinaus sind auch mehrere Kopplungselemente einer Lanzette denkbar, so dass eine Selektion einer Spitze durch die Auswahl eines entsprechenden Kopplungselementes erfolgt. In den beschriebenen Ausführungsformen ist dann z.B. das Kopplungselement der Lanzette gleichzeitig ein Bestandteil eines Mittels zur Selektion, wie es bereits schon mehrfach dargestellt wurde. Allgemein zeigt sich somit, dass in Abhängigkeit der jeweiligen Ausgestaltung eines Systems/ Vorrichtung, die zur Verwendung der erfindungsgemäßen Lanzette geeignet sind, vielfältige Ausführungsformen eines Kopplungselementes denkbar sind, die Vorteilhafterweise gleichzeitig eine Selektion einer Lanzettenspitze ermöglichen. Weiterhin kann die erfindungsgemäße Lanzette zusätzlich Mittel zur Selektion beinhalten, wie sie bereits schon beschrieben wurden. Beispielsweise seien hier Markierungen der jeweiligen Lanzettenspitzen genannt, die in einer entsprechenden Vorrichtung eine gezielte Selektion einer Lanzettenspitze z.B. mittels einer optischen Detektion der Markierung erlauben und damit eine Auswahl der Einstichtiefe zulassen. Vorteilhafterweise sind somit bei derartigen Systemen die Mittel zur Selektion sowohl Bestandteil der Vorrichtung als auch der Lanzette selber, wobei ein entsprechendes Zusammenwirken zwischen Vorrichtung und Lanzette eine Selektion einer Lanzettenspitze ermöglicht.

Wie bereits erwähnt, kann in einem System zur Gewinnung von Körperflüssigkeit die Vorrichtung mit weiteren verschiedenen Elementen zur Analyse einer Körperflüssigkeit kombiniert werden (z.B. Testelement, Detektor, Auswerteeinheit etc.). Beispielhaft wird eine System mit einem Trägerband beschrieben, das aber nicht einschränkend auf die Auswahl der Elemente in einem System zu lesen ist. Dieses System besteht bevorzugter weise aus einem Gehäuse, in dem ein im Wesentlichen planares Trägerband montiert ist, und mindestens zwei Lanzetten, die liegend auf dem Trägerband angeordnet sind. Das Gehäuse weist mindestens eine Öffnung auf, auf die die Lanzette beim Stechvorgang zu bewegt wird und wenn nötig, hindurch treten kann. Das im Wesentlichen planare Trägerband ist bevorzugter weise auf zwei Spulen aufgewickelt. Es können aber auch andere Bevorratungsmöglichkeiten zur Magazinierung der benutzten und unbenutzten Lanzetten eingesetzt werden. Bei Verwendung von zwei Spulen zur Magazinierung der Lanzetten befinden sich die unbenutzten Lanzetten auf der einen Spule und die benutzten Lanzetten auf der anderen Spule.

Die Lanzetten bestehen aus einem Material, das weich genug ist, um auf dem Trägerband aufgewickelt zu werden, ohne dabei geknickt zu werden. Andererseits ist das Material der Lanzetten so stabil, dass die Lanzette bei Aktuation und Eintritt in die Haut nicht verformt wird. Alternativ werden die Lanzetten quer auf dem Trägerband angeordnet, so dass ein Biegen der Lanzette vermieden werden kann. Eine weitere Möglichkeit ein Biegen der unbenutzten Lanzetten zu vermeiden, ist die Wahl des Durchmessers der Spule auf dem die Lanzetten aufbewahrt werden, sodass die Lanzetten beim Aufrollen kaum gebogen werden.

Die Lanzette weist mindestens zwei Lanzettenspitzen auf, die sich am Lanzettenkörper befinden. Nachdem der Benutzer die Stechtiefe gewählt hat, wird durch das Mittel der Selektion (in diesem Fall ein Knickelement) die Lanzettenspitze ur Benutzung bereit gestellt. In dieser bevorzugten Ausführungsform wird dis Lanzette folglich durch Abknicken selektiert. Durch das Knickelement, das auf die Lanzette so einwirkt, ist die Lanzettenspitze in Bezug auf den restlichen Lanzettenkörper in ihrer Ausrichtung veränderbar ist. In einer bevorzugten Ausführungsform kann das Knickelement bei Krafteinwirkung auf die Lanzette vor der Aktuation, den Ort der Krafteinwirkung auf den Lanzettenkörper regeln. Hierzu kann das Knickelement durch ein Regelelement gesteuert werden. Zur Kraftübertragung kann ein Stößel bzw. Umlegehebel dienen. In dem System können herkömmliche Lanzetten benutzt werden, bevorzugter weise sind die Lanzetten als Flachlanzetten ausgeprägt.

Die Lanzette wird von einem Antriebselement in Richtung Gehäuseöffnung bewegt, um dort den Stechvorgang auszuüben. Bei der abgeknickten Lanzettenspitze kann dies nach oder während dieses Knickvorgangs stattfinden. Dabei bewegt sich mindestens ein Teil der Lanzette in Richtung der Gehäuseöffnung und sticht in die Haut des Patienten. An der Einstichstelle bildet sich ein Bluttropfen, der zur Analyse benutzt wird. Wenn sich ein Testelement auf dem Trägerband befindet, wird das Trägerband, wenn nötig so weit transportiert, dass des Testelement sich unterhalb der Gehäuseöffnung befindet. Der Bluttropfen kann auf das Testelement aufgebracht werden, ohne dass der Patient weitere Schritte einleiten muss. Das Blut reagiert mit einem oder mehreren Reagenzien, die sich auf dem Testelement befinden, wie sie z. B. aus den Dokumenten EP-A 0 885 591, EP-B 0 535 480 und EP-B 0 477 322 bekannt sind. Das Testelement wird mittels eines Detektors analysiert.

Das Blut kann auf verschiedene Komponenten hin untersucht werden, wie es im Stand der Technik bekannt ist. Zum Beispiel kann die Analyse auf Blutbestandteile wie Hämatokrit, Glucose, Cholesterin, Koagulation, Eisen und andere gerichtet sein. Zur Analyse können unterschiedliche Methoden zur Anwendung kommen. So können beispielsweise elektrochemische Nachweisreaktionen benutzt werden, aber auch optische (z.B. Reflektion, Absorption, Fluoreszenz, Raman-Spektroskopie) oder magnetische Nachweisreaktionen. Typischerweise wird die Flüssigkeit mit einem Testsystem in Kontakt gebracht, wobei eine Reaktion zwischen einem Testelement und der Flüssigkeit stattfindet. So beruht die Detektion mittels eines optischen Testelements auf einer Farbreaktion zwischen Flüssigkeit und Nachweisreagenz. Beispiele für diese Reaktionen sind in den US Patenten 3,802,842; 4,061,468 und 4,490,465 beschrieben.

Bei der Benutzung des Gerätes führt das System verschiedene Schritte durch. Am Gerät wird eine Stechtiefe gewählt. Diese Auswahl führt dazu, dass das System nach einem zuvor beschriebenen Auswahlmechanismus mit Hilfe des Mittels zur Selektion eine Lanzette auswählt, die der vorgewählten Stechtiefe entspricht. Wenn die Lanzette vor dem Einstich geknickt werden soll, wird sie in eine Position gebracht, in der sie durch Einwirkung einer Schwellenkraft auf den Lanzettenkörper in den geknickten Zustand gebracht wird. Dabei wird vorzugsweise der Sterilschutz durchbrochen. Wird die Lanzette vor dem Einstich nicht abgeknickt, so wird der Sterilschutz vor oder während des Einstichs entfernt. Die Lanzette wird, wenn nötig, bis zur Öffnung des Gehäuses transportiert. Dort wird sie mit Hilfe eines Antriebselements aktuiert und kann dabei zu einem Teil aus der Gehäuseöffnung austreten. Bei dem Aktuationsvorgang tritt die Lanzette zumindest zu einem Teil in die Haut des Patienten ein und danach wieder in das Gerät zurück.

Befindet sich die Lanzette auf einem Trägerband, so kann dieses Trägerband weiter transportiert werden und auf die zweite Spule gewickelt werden. Dort liegt die Lanzette wieder flach auf dem Trägerband. Dieser Remagazinierungsvorgang wird in der Patentanmeldung US 20050245845 beschrieben.

In einem integrierten System, in dem auch Testelemente auf dem Trägerband, vorzugsweise alternierend mit den Lanzetten aufgebracht sind, wird das Testelement nach dem Stechvorgang bis zur Gehäuseöffnung transportiert, um den Bluttropfen zur Analyse aufzunehmen. Das Testelement kann bis zum Detektor transportiert und dort vermessen werden.

### Figurenbeschreibung

- Figur 1:: Schematische Darstellung eines Lanzettenkörpers mit mehreren Lanzettenspitzen unterschiedlicher Länge, die auf zwei Seiten des Lanzettenkörpers angebracht sind.
- Figur 2:: Schematische Darstellung einer sternförmigen Anordnung der Lanzettenspitzen an einem Lanzettenkörper.
- Figur 3:: Schematische Darstellung einer abknickbaren Spitze an dem Lanzettenkörper.
- Figur 4:: Schematische Darstellung eines Microsamplers mit Knickstelle an einem Lanzettenkörper
- Figur 5:: Schematische Darstellung eines Microsamplers mit eingeprägtem Knickbereich an einem Lanzettenkörper
- Figur 6:: Schematische Darstellung eines Systems mit einem Trägerband, auf dem Lanzetten angebracht sind, mit Selektionsmittel, Stechmittel und Optik.
- Figur 7a:: Schematische Darstellung einer kreisförmigen Anordnung von verschiedenen Lanzetten, die unterschiedliche Längen bzw. unterschiedliche Knickbereiche aufweisen.
- Figur 7b:: Schematische Darstellung eines Lanzettenarmes mit einer Schneide am distalen Ende der Lanzette.
- Figur 8 a-d:: Schematische Darstellung eines Knickvorgangs vor dem Einstichvorgang mit Hilfe einer Knickvorrichtung, die ebenfalls ein Rückbiegeelement enthält.
- Figur 9:: Schematische Darstellung eines Lanzettenrades, das mit Excenterwalzen bewegt wird.

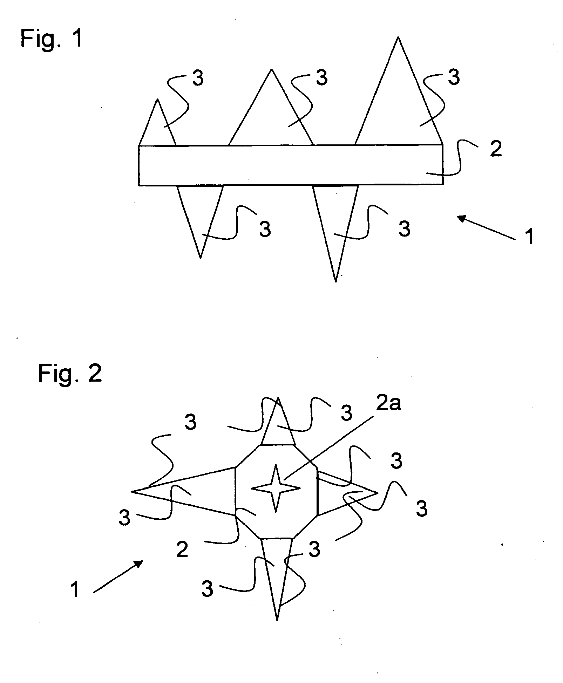

In Figur 1 ist eine Lanzette (1) dargestellt, die aus einem Lanzettenkörper (2) und mindestens 2 Lanzettenspitzen (3) besteht. In der in Figur 1 gezeigten Anordnung befinden sich ein Teil der Lanzettenspitzen (3) auf einer Seite des hier rechteckig dargestellten Lanzettenkörpers (2) und der restliche Teil der Lanzettenspitzen (3) auf der gegenüberliegenden Seite des Lanzettenkörpers (2). In solch einer bandähnlichen Struktur des Lanzettenkörpers (2) können mehrere 100 Lanzetten angeordnet sein. Bevorzugterweise sind bis zu 100 Lanzettenspitzen (3) dem Lanzettenkörper angeordnet, die dann beispielsweise auf einem Band aufgewickelt werden können. In einer weiteren Ausführungsform sind bis zu 6 Lanzettenspitzen (3) an dem Lanzettenkörper (2) angeordnet, wobei die Lanzettenkörper dann stapelförmig in einem Magazin angeordnet werden können. Bei der Verwendung eines Trägerbandes zur Magazinierung der Lanzettenspitzen, ist es auch möglich, dass das Trägerband als Lanzettenkörper dient, an dem die Lanzettenspitzen angeordnet sind. Dieses Trägerband kann beispielsweise aus Stoff bestehen, oder aber auch ein Metallband sein. Das Mittel zur Selektion, also die Auswahl der Lanzettenlänge und damit der Stechtiefe, kann hierbei ein Vorrichtungselement sein, das zum Weitertakten der Vorrichtung dient. Ein genau eingestellter Mechanismus befördert das Band bei jedem Taktvorgang von einer zur nächsten Lanzettenspitze. Hierzu müssen die Lanzettenspitzen präzise angeordnet sein. Bei jedem Weitertakten wird der Lanzettenkörper um eine Lanzettenspitze weiter verschoben. Dies kann dem Benutzer über einen Zähler angezeigt werden, sodass für den Benutzer immer ersichtlich ist, welche Lanzettenlänge gerade zum Einstich bereit gestellt ist. Dabei sollte verhindert werden, dass die Vorrichtung zurück getaktet wird, damit keine wiederholte Benutzung einer Lanzettenspitze möglich ist.

In Figur 2 sind die Lanzettenspitzen (3) in verschiedenen Winkeln von den Lanzettenkörpern (2) angeordnet. Die Lanzettenspitzen (3) können hierbei in alle Raumrichtungen weisen. Bevorzugterweise sind die Lanzettenspitzen (3) in einer Ebene angeordnet, so dass die Lanzette (1) in einem Stapelmagazin bevorratet werden kann, ohne dass die Lanzettenspitzen beschädigt werden. In dieser sternförmigen Anordnung können, je nach Geometrie des Lanzettenkörpers bis zu 10 Lanzetten in verschiedenen Winkeln an den Grundkörper angeordnet sein, bevorzugterweise sind das 4 bis 5 Lanzettenspitzen (3). Vorteilhafterweise weist der Lanzettenkörper eine Ausnehmung / Loch (2a) auf, das als Kopplungselement zum Ankoppeln an einen entsprechend ausgestalteten Stößel (nicht gezeigt) einer Antriebseinheit vorgesehen ist. Im gezeigten Beispiel wird die Lanzette drehbar mit der Antriebseinheit verbunden, so dass aufgrund einer Rotation des Stößels und somit der Lanzette eine gewünschte Spitze für einen Stechvorgang ausgewählt werden kann. Auf diese Weise ist aufgrund des Zusammenwirkens der Antriebseinheit mit dem Kopplungselement der Lanzette eine Selektion einer Lanzettenspitze möglich.

In einer bevorzugten Ausführungsform weisen die Lanzettenspitzen (3) an ihrem proximalen Ende (3a), der an den Lanzettenkörper (2) grenzt eine Knickstelle auf. Durch diese Knickstelle kann die Lanzettenspitze (3) aus der Ebene des Grundkörpers und der restlichen Lanzettenspitzen herausgeknickt werden, wodurch eine Selektion der gewünschten Lanzettenspitze (3) erfolgen kann. Beim Stechvorgang wird mit der gesamten Lanzette (1) der Stechvorgang vorgenommen.

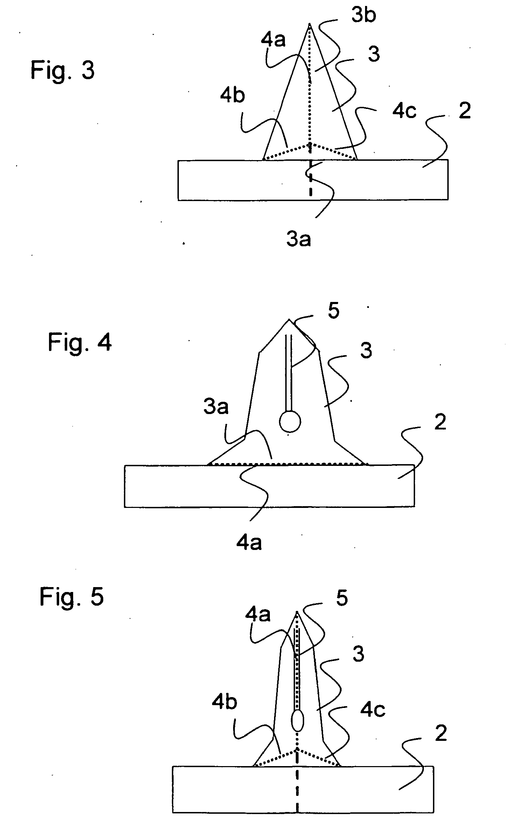

In Figur 3 ist ein Ausschnitt einer Lanzette (1) gezeigt, wobei die hier gezeigte Lanzettenspitze (3), die sich am Lanzettenkörper (2) befindet, einen Bereich mit mehreren Knicklinien (4a, b, c) aufweist. Die Knicklinie (4a) erstreckt sich von dem distalen Ende 3b der Lanzettenspitze(3) in Richtung proximalen Ende (3a) der Lanzettenspitze (3). Diese Knicklinie (4a) kann sich dabei über den kompletten Spitzenbereich der Lanzettenspitze (3) erstrecken oder nur über einen Teilbereich der Lanzettenspitze (3).

Weitere Knicklinien (4b und 4c) können sich seitlich von der ersten Knicklinie ab in Richtung proximalem Ende (3a) der Lanzettenspitze (3) erstrecken.

Die Figur 4 zeigt eine Lanzettenspitze (3) mit einem integrierten Microsamplers (5). Die Lanzettenspitze (3) mit integrierten Microsampler (5) kann über eine Knicklinie (4a) am proximalen Ende (3a) der Lanzettenspitze (3) abgeknickt werden.

Figur 5 zeigt eine besondere Ausführungsform der Lanzettenspitze (3) mit integriertem Microsampler (5), wobei sich wie in Figur 3 mehrere Knicklinien (4a 4b und 4c) befinden.

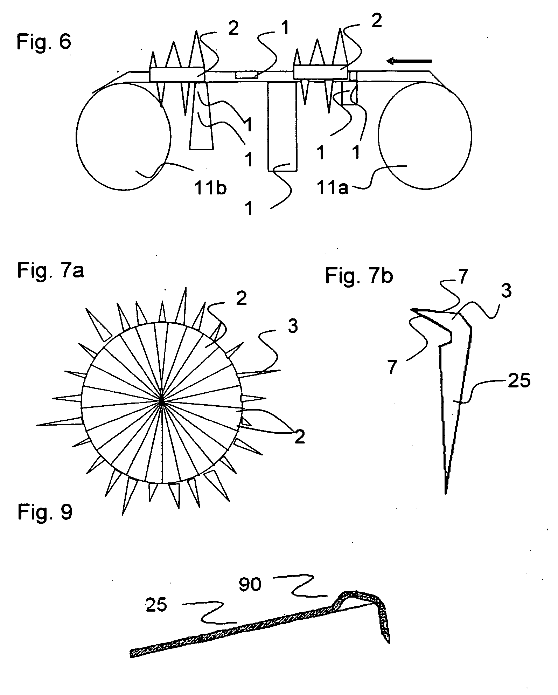

Figur 6 zeigt ein System mit einem Trägerband (10), das auf zwei verschiedenen Rollen (11a und 11b) aufgewickelt ist, wobei eine der Rollen (11a) unbenutzt Lanzetten (1) bzw. Testelemente (13) enthalten kann, während Rolle (11b) benutzte Lanzetten (1) bzw. Testelemente magaziniert. Das System kann weiterhin ein Knickelement (12) aufweisen, das nach Auswahl der entsprechenden Lanzettenspitze (3), die Lanzette abknickt. In diesem Fall dient das Knickelement als Mittel zur Selektion Des Weiteren kann das System eine Optik sowie Mittel zum Antrieb der Lanzette, wie einen Stößel enthalten. Auch die Optik kann als Mittel zur Selektion dienen, wenn diese als Sensor für eine Markierung auf dem Trägerband (10) oder dem Lanzettenkörper (2) mit Lanzettenspitzen (3) ausgestaltet ist

Figur 7a zeigt eine kreisförmige Anordnung von Lanzettenspitzen (3) und Lanzettenträgern (23) in Form eines Lanzettenrades (24). Hierbei können die Lanzettenspitzen (3) zu dem Lanzettenträgern (23) abknickbar sein und dabei auch unterschiedliche Längen aufweisen. Die Lanzettenträger (23) können dabei durch Furchen voneinander getrennt sein, so dass jeder Lanzettenarm (25) aus der Ebene der kreisförmigen Lanzette (1) ausgelenkt werden kann. Dies kann zur Ausführung des Stechvorgangs benutzt werden. Dabei vollzieht der Lanzettenarm (25) beim Einstich wiederum eine Kreisbewegung. Der Lanzettenarm (25) kann dabei Stege aufweisen, die die Einstichtiefe begrenzen, da sie beim Einstich in den Körper ein weiteres Eindringen der Lanzette verhindern. Durch eine entsprechende Vorbiegung der Lanzettenarme (25) bewegt sich die Lanzette nach dem Einstich von alleine wieder in die ursprüngliche Position zurück, wie dies auch in Figur 9 gezeigt ist Der Lanzettenarm (25) kann aufgrund der Flexibilität sowohl nach oben als auch nach unten ausgelenkt werden. Da der Lanzettenarm (25) bei der Einstichbewegung eine Kreisbewegung vollzieht, wird der Körper des Benutzers nicht senkrecht angestochen sondern in einer Kreisbewegung. Dies hat zur Folge, dass nicht nur Kraft senkrecht auf die Haut ausgeübt wird sondern auch laterale Kräfte wirken. Dadurch ist der Schmerz beim Einstich größer als bei einer herkömmlichen Lanzette, die senkrecht in die Haut eingestochen wird. Um diese lateralen Kräfte beim Stechvorgang zu verringern, kann die Lanzettenspitze (3) der Lanzette (1) eine Schneide (70) besitzen, die die Haut des Körpers aufschneidet und nicht wie bei herkömmlichen Lanzetten die Haut durchsticht.

In Figur 7b ist eine Lanzette (1) dargestellt, die eine Schneide an ihrem distalen Ende (3b) aufweist. Die Schneide weist im Gegensatz zu einer Lanzettenspitze (3), (wie in den vorhergehenden Figuren gezeigt) keine gleichförmig aufeinander zu laufenden Kanten auf, die sich in der Spitze (3) vereinigen. Bei der Schneide laufen die Kanten und der Schneide in unterschiedlichen Winkeln auf die Spitze zu. Je nach Anordnung der Schneide zum Lanzettenkörper (25) können die erste Seitenkante und die zweite Seitenkante in der Ebene der Kreisbahn verlaufen oder quer dazu. Sind die Seitenkanten in der Ebene der Kreisbahn angeordnet, do können die geschliffene Seitenkanten in Bewegungsrichtung schneiden, während die quer zur Kreisbahn angeordneten Seitenkanten auch quer zur Bewegungsrichtung schneiden, was zu mehr Schmerz beim Einstich führen kann. Ein ähnlich ausgestaltetes Rad, wie das Lanzettenrad (24), kann auch für Testelemente ausgestaltet werden, das zusammen mit dem Lanzettenrad (24) in einem System so angeordnet sein kann, dass Einstich und Blutübertrag an einer Öffnung durchgeführt werden kann.

In den Figuren 8 a-d ist eine Vorrichtung schematisch dargestellt, die das Abknicken vor dem Einstich und das Zurückbiegen nach dem Einstich gewährleistet. Diese Vorrichtung ist bevorzugt mit Lanzetten zu verwenden, die auf einem Trägerband (10) angebracht sind. Sie kann aber auch für Lanzetten verwendet werden, die nicht auf einem Trägerband (10) angebracht sind. Vor dem Einstichvorgang soll die Lanzette (1) aus der Trägerbandebene herausgeknickt werden. Dieser Zustand ist in Figur 8a dargestellt. Hierzu wird das Trägerband (10) zwischen dem Unterteil (82) sowie dem Oberteil (81) des Halteelementes (80) und dem Schieber (89) eingeklemmt. Hierdurch wird ein Teil des Trägerbandes (10) umgeknickt und dadurch wird ein Verrutschen während des Knickvorgangs verhindert. Anschließend wird mit Hilfe eines Umlegeelementes (84), das durch einen Stößel (85) angetrieben wird die Lanzette (1) an einer vordefinierten Stelle geknickt. Das Umlegeelement (84) übt bei diesem Vorgang soviel Druck auf die Lanzette aus, dass sie an der Stelle, an der sie das Oberteil (81) des Halteelementes (80) berührt abknickt, wie in Figur 8b gezeigt. Wie in Figur 8c gezeigt, wird nach dem Knickvorgang die Halterung des Trägerbandes (10) durch das Halteelement (80) gelöst und das Halteelement (80) kann aus der Trägerbandebene herausgedreht werden, um den Stechvorgang nicht zu behindern. Die Lanzette (1) kann durch Bewegen des Schiebers zum Einstich bewegt werden. Um die Einstichtiefe beim Stechvorgang beeinflussen zu können, ist das Halteelement (80) mit einem Schlitten (88) verbunden, der das Halteelement (80), das hier als Bezugselement für die Einstellung der Stechtiefe dient, verschieben zu können. Durch das Verschieben des Halteelementes (80) schiebt sich das Oberteil (81) des Halteelementes (80) mehr oder weniger weit über das Trägerband (10) hin zur Lanzettenspitze. Durch dieses Verschieben ist die Lanzettenspitze nach dem Abknickvorgang unterschiedlich lang, je nachdem wie weit das Halteelement (80) vor oder zurückgeschoben wird. Das Verschieben des Schlittens (88) kann bevorzugter weise durch ein Verschiebegewinde (87) geschehen. Zusätzlich kann an dem Halteelement (80) ein Rückbiegeelement (86) angebracht sein, dass nach dem Einstich der Lanzette (1) in den Körper, die Lanzette (1) wieder in die Trägerbandebene zurück biegt.

Figur 9 zeigt einen Lanzettenarm (25) der eine Wölbung (93) aufweist, die leicht aus der Ebene des Lanzettenarmes (25) herausragt. In dieser Wölbung kann sich der Kanal des Microsamplers befinden. Hierdurch kann auf einfache Weise ein Testelement an die Wölbung herangeführt werden, um das Blut, das in dem Kanal des Microsamplers gesammelt wurde zu übertragen.

### Bezugszeichenliste

- 1: Lanzette
- 2: Lanzettenkörper
- 3: Lanzettenspitze
- 3 a: proximales Ende
- 3 b: distales Ende
- 4 a: 1. Knicklinie
- 4 b: 2. Knicklinie
- 4 c: 3. Knicklinie
- 5: Microsamplers
- 10: Trägerband
- 11: a 1. Rolle
- 11b: 2. Rolle
- 23: Lanzettenträger
- 24: Lanzettenrad
- 25: Lanzettenarm
- 80: Halteelement
- 81: Oberteil Halteelement
- 82: Unterteil Halteelement
- 83: Sterilschutz
- 84: Umlegeelement
- 85: Stößel
- 86: Rückbiegeelement
- 87: Verschiebegewinde
- 88: Schlitten
- 89: Schieber
- 90: Wölbung

## Patentansprüche

1. Vorrichtung zur Gewinnung von Körperflüssigkeit, beinhaltend:
mindestens eine Lanzette (1), beinhaltend einen Lanzettenkörper (2) mit mindestens zwei Spitzen (3), die unterschiedliche Längen aufweisen, sowie
eine Antriebseinheit zum Bewegen der Lanzette
**dadurch gekennzeichnet, dass** die Vorrichtung ein Mittel zur Selektion besitzt, mit dessen Hilfe eine Spitze (3) ausgewählt wird, wobei das Mittel zur Selektion es ermöglicht, den Lanzettenkörper so zu positionieren, dass die ausgewählte Lanzettenspitze zur Benutzung bereitgestellt wird, sodass sie mit der Antriebseinheit Wechsel wirken kann, um zum Einstich bewegt zu werden, so dass ein Stechvorgang mit definierter Einstichtiefe in ein gegen die Vorrichtung angelegtes Körperteil, in Abhängigkeit von der Länge der ausgewählten Spitze (3) erfolgt, wobei mindestens eine der nicht ausgewählten Spitzen derartig in der Vorrichtung während eines Stechvorgangs positioniert wird, dass mit der nicht ausgewählten Spitze kein Stechvorgang ausgeführt wird.

2. Vorrichtung nach Anspruch 1, **dadurch gekennzeichnet, dass** die mindestens zwei Spitzen in verschiedene Richtungen am Lanzettenkörper angeordnet sind.

3. Vorrichtung nach Anspruch 1 oder 2, **dadurch gekennzeichnet, dass** die mindestens zwei Spitzen sternförmig am Lanzettenkörper angeordnet sind.

4. Vorrichtung nach Anspruch 1, **dadurch gekennzeichnet, dass** die mindestens zwei Spitzen linear am Lanzettenkörper angeordnet sind.

5. Vorrichtung nach einem der Ansprüche 1 bis 4, **dadurch gekennzeichnet, dass** die Vorrichtung eine Knickvorrichtung besitzt, die die Veränderung der Ausrichtung der Spitzen gegenüber dem Lanzettenkörper bewirkt.

6. Vorrichtung nach einem der Ansprüche 1 bis 5, **dadurch gekennzeichnet, dass** mindestens eine Spitze in ihrer Ausrichtung veränderbar gegenüber dem Lanzettenkörper ist.

7. Vorrichtung nach einem der Ansprüche 1 bis 6, **dadurch gekennzeichnet, dass** die mindestens eine Lanzette eine Flachlanzette ist.

8. Vorrichtung nach einem der Ansprüche 1 bis 7, **dadurch gekennzeichnet, dass** die mindestens zwei Spitzen einen Knickbereich aufweisen, der mindestens eine Struktur mit einer geänderten Steifigkeit besitzt.

9. Vorrichtung nach einem der Ansprüche 1 bis 8, **dadurch gekennzeichnet, dass** sich mindestens eine Struktur mit geänderter Steifigkeit über einen Teil der Längsausdehnung des Spitzenbereiches erstreckt.

10. Vorrichtung nach einem der Ansprüche 8 oder 9, **dadurch gekennzeichnet, dass** bei Einwirkung einer Schwellenkraft auf die Lanzette, die mindestens eine Struktur mit veränderter Steifigkeit, ein Abknicken der angrenzenden Flächen bewirkt.

11. Vorrichtung nach einem der Ansprüche 1 bis 10, **dadurch gekennzeichnet, dass** die Lanzette auf einem Trägerband angeordnet ist.

12. Vorrichtung nach Anspruch 11, **dadurch gekennzeichnet, dass** die mindestens eine Lanzette in verschiedenen Ausrichtungen auf dem Trägerband angeordnet ist.

13. Vorrichtung nach Anspruch 11 oder 12, **dadurch gekennzeichnet, dass** auf dem Trägerband weiterhin mindestens ein Testelement angeordnet ist.

14. Vorrichtung nach einem der Ansprüche 11 bis 13, **dadurch gekennzeichnet, dass** die Lanzette an ihrem proximalen Ende auf dem Trägerband fixiert ist.

15. Vorrichtung nach einem der vorherigen Ansprüche, **dadurch gekennzeichnet, dass** mindestens die Lanzettenspitzen einen Sterilschutz besitzen.

16. Vorrichtung nach einem der vorherigen Ansprüche, **dadurch gekennzeichnet, dass** die Vorrichtung ein Mittel zum Zurückknicken der geknickten Spitzen besitzt.

17. Vorrichtung nach einem der vorherigen Ansprüche, **dadurch gekennzeichnet, dass** die Vorrichtung ein Testelement aufweist

18. Vorrichtung nach einem der vorherigen Ansprüche, **dadurch gekennzeichnet, dass** die Vorrichtung eine Detektionseinheit aufweist.

19. Lanzette (1) zum Einstechen in ein Körperteil beinhaltend:
einen Lanzettenkörper (2) mit mindestens zwei Spitzen (3), die unterschiedliche Längen aufweisen, und
die Lanzette (1) derartig ausgestaltet ist, dass eine Spitze (3) von den mindestens zwei Spitzen auswählbar ist, so dass ein Stechvorgang mit definierter Einstichtiefe in Abhängigkeit von der Länge der ausgewählten Spitze erfolgen kann, und die ausgewählte Lanzettenspitze und die weiteren Lanzettenspitzen so angeordnet sind, dass nur die ausgewählte Lanzettenspitze während eines Stechvorgangs in den zu stechenden Körper eindringen kann und mindestens eine der nicht ausgewählten Spitzen derartig während eines Stechvorgangs relativ zu der ausgewählten Spitze positioniert ist, dass mit der nicht ausgewählten Spitze kein Stechvorgang ausgeführt wird,
**dadurch gekennzeichnet, dass** der Lanzettenkörper (2) ein Trägerband ist oder dass der Lanzettenkörper (2) auf einem Trägerband fixiert ist.

20. Lanzette nach Anspruch 19, **dadurch gekennzeichnet, dass** durch einen Knickbereich in der Lanzette eine Spitze auswählbar ist.

21. Lanzette nach Anspruch 19 oder 20, **dadurch gekennzeichnet, dass** die Lanzette ein Kopplungselement aufweist, durch das die Lanzette an einen Antriebseinheit eines Blutentnahmesystems angekoppelt werden kann.

22. Lanzette nach Anspruch 21, **dadurch gekennzeichnet, dass** das Kopplungselement eine Ankopplung der Lanzette in verschiedenen Positionen relativ zur Antriebseinheit erlaubt.

23. Lanzette nach Anspruch 21, **dadurch gekennzeichnet, dass** die Lanzette mehrere Kopplungselemente aufweist.

24. Lanzette nach Anspruch 21 **dadurch gekennzeichnet, dass** die Lanzettenspitzen am Lanzettenkörper unterschiedliche Ausrichtungen aufweisen.

## Claims

1. Device for collecting body fluid comprising:
at least one lancet (1) comprising a lancet body (2) with at least two tips (3) which have different lengths, and
a drive unit for moving the lancet
**characterized in that** the device has a selection means with the aid of which a tip (3) is selected wherein the selection means enables the lancet body to be positioned in such a manner that the selected lancet tip is made available for use so that it can interact with the drive unit in order to be moved for the puncture such that a lancing process takes place with a defined lancing depth into a body part resting against the device, depending on the length of the selected tip (3) where at least one of the unselected tips is positioned in the device during a lancing process in such a manner that no lancing process is executed with the unselected tip.

2. Device according to claim 1, **characterized in that** the at least two tips are arranged in different directions on the lancet body.

3. Device according to claim 1 or 2, **characterized in that** the at least two tips are arranged in a star shape on the lancet body.

4. Device according to claim 1, **characterized in that** the at least two tips are arranged linearly on the lancet body.

5. Device according to one of the claims 1 to 4, **characterized in that** the device has a bending device which changes the alignment of the tips relative to the lancet body.

6. Device according to one of the claims 1 to 5, **characterized in that** the alignment of at least one tip can be changed relative to the lancet body.

7. Device according to one of the claims 1 to 6, **characterized in that** the at least one lancet is a flat lancet.

8. Device according to one of the claims 1 to 7, **characterized in that** the at least two tips have a bending region which has at least one structure with an altered stiffness.

9. Device according to one of the claims 1 to 8, **characterized in that** at least one structure with altered stiffness extends over a part of the longitudinal extension of the tip region.

10. Device according to one of the claims 8 or 9, **characterized in that** when a threshold force acts on the lancet, the at least one structure with an altered stiffness results in a bending of the adjoining areas.

11. Device according to one of the claims 1 to 10, **characterized in that** the lancet is arranged on a carrier tape.

12. Device according to claim 11, **characterized in that** the at least one lancet is arranged in different orientations on the carrier tape.

13. Device according to claim 11 or 12, **characterized in that** at least one test element is additionally arranged on the carrier tape.

14. Device according to one of the claims 11 to 13, **characterized in that** the proximal end of the lancet is attached to the carrier tape.

15. Device according to one of the previous claims, **characterized in that** at least the lancet tips have a sterile protection.

16. Device according to one of the previous claims, **characterized in that** the device has a means for bending back the bent tips.

17. Device according to one of the previous claims, **characterized in that** the device has a test element.

18. Device according to one of the previous claims, **characterized in that** the device has a detection unit.

19. Lancet (1) for insertion into a body part, comprising:
a lancet body (2) with at least two tips (3) which have different lengths, and
the lancet (1) is designed such that one tip (3) of the at least two tips can be selected such that a lancing process can take place with a defined lancing depth depending on the length of the selected tip and the selected lancet tip and the other lancet tips are arranged such that only the selected lancet tip can penetrate into the body to be punctured during a lancing process and at least one of the unselected tips can be positioned relative to the selected tip during a lancing process in such a manner that no lancing process is executed with the unselected tip,
**characterized in that** the lancet body (2) is a carrier tape or that the lancet body (2) is attached to a carrier tape.

20. Lancet according to claim 19, **characterized in that** a tip can be selected by means of a bending region in the lancet.

21. Lancet according to claim 19 or 20, **characterized in that** the lancet has a coupling element which allows the lancet to be coupled to a drive unit of a blood collection system.

22. Lancet according to claim 21, **characterized in that** the coupling element allows the lancet to be coupled in different positions relative to the drive unit.

23. Lancet according to claim 21, **characterized in that** the lancet has a plurality of coupling elements.

24. Lancet according to claim 21, **characterized in that** the lancet tips on the lancet body have different orientations.

## Revendications

1. Dispositif d'obtention de liquide corporel, comportant :
au moins une lancette (1) comprenant un corps de lancette (2) doté d'au moins deux pointes (3) présentant des longueurs différentes, ainsi qu'une unité d'entraînement pour déplacer la lancette,
**caractérisé en ce que** le dispositif possède un moyen de sélection à l'aide duquel une pointe (3) est sélectionnée, ledit moyen de sélection permettant de positionner le corps de lancette de façon à ce que la pointe de lancette sélectionnée soit mise à disposition pour l'emploi de sorte qu'elle puisse interagir avec l'unité d'entraînement pour être déplacée vers la piqûre, permettant ainsi d'effectuer une piqûre de profondeur définie dans une partie du corps appliquée contre le dispositif, en fonction de la longueur de la pointe sélectionnée (3), au moins une des pointes non sélectionnées étant positionnée de telle façon dans le dispositif pendant une piqûre que la pointe non sélectionnée ne puisse être utilisée pour effectuer une piqûre.

2. Dispositif selon la revendication 1, **caractérisé en ce que** lesdites au moins deux pointes sont disposées selon des directions différentes sur le corps de lancette.

3. Dispositif selon la revendication 1 ou 2, **caractérisé en ce que** lesdites au moins deux pointes sont disposées en étoile sur le corps de lancette.

4. Dispositif selon la revendication 1, **caractérisé en ce que** lesdites au moins deux pointes sont disposées de façon linéaire sur le corps de lancette.

5. Dispositif selon une des revendications 1 à 4, **caractérisé en ce que** le dispositif possède un dispositif de pliage ayant pour effet de modifier l'orientation des pointes par rapport au corps de lancette.

6. Dispositif selon une des revendications 1 à 5, **caractérisé en ce qu'**au moins une pointe est modifiable quant à son orientation par rapport au corps de lancette.

7. Dispositif selon une des revendications 1 à 6, **caractérisé en ce que** ladite au moins une lancette est une lancette plate.

8. Dispositif selon une des revendications 1 à 7, **caractérisé en ce que** lesdites au moins deux pointes présentent une zone pliée possédant au moins une structure de rigidité modifiée.

9. Dispositif selon une des revendications 1 à 8, **caractérisé en ce qu'**au moins une structure de rigidité modifiée s'étend sur une partie de l'extension longitudinale de la zone de pointe.

10. Dispositif selon une des revendications 8 ou 9, **caractérisé en ce que** ladite au moins une structure de rigidité modifiée a pour effet une pliure des surfaces adjacentes.

11. Dispositif selon une des revendications 1 à 10, **caractérisé en ce que** la lancette est disposée sur une bande support.

12. Dispositif selon la revendication 11, **caractérisé en ce que** ladite au moins une lancette est disposée selon des orientations différentes sur la bande support.

13. Dispositif selon la revendication 11 ou 12, **caractérisé en ce qu'**au moins un élément de test est encore disposé sur la bande support.

14. Dispositif selon une des revendications 11 à 13, **caractérisé en ce que** la lancette est fixée à son extrémité proximale sur la bande support.

15. Dispositif selon une des revendications précédentes, **caractérisé en ce qu'**au moins les pointes de lancette possèdent une protection stérile.

16. Dispositif selon une des revendications précédentes, **caractérisé en ce que** le dispositif possède un moyen permettant de replier les pointes pliées.

17. Dispositif selon une des revendications précédentes, **caractérisé en ce que** le dispositif présente un élément de test.

18. Dispositif selon une des revendications précédentes, **caractérisé en ce que** le dispositif présente une unité de détection.

19. Lancette (1) destinée à piquer une partie du corps, comportant:
un corps de lancette (2) doté d'au moins deux pointes (3) présentant des longueurs différentes,
la lancette (1) étant conçue de telle façon qu'une pointe (3) puisse être sélectionnée parmi lesdites au moins deux pointes, permettant ainsi d'effectuer une piqûre de profondeur définie en fonction de la longueur de la pointe sélectionnée, et la pointe de lancette sélectionnée ainsi que les autres pointes de lancette étant disposées de telle manière que seule la pointe de lancette sélectionnée puisse pénétrer dans le corps à piquer pendant une piqûre, et au moins une des pointes non sélectionnées est positionnée pendant une piqûre de telle façon par rapport à la pointe sélectionnée que la pointe non sélectionnée ne puisse être utilisée pour effectuer une piqûre,
**caractérisée en ce que** le corps de lancette (2) est une bande support ou en ce que le corps de lancette (2) est fixé sur une bande support.

20. Lancette selon la revendication 19, **caractérisée en ce qu'**une zone pliée prévue dans la lancette permet de sélectionner une pointe.

21. Lancette selon la revendication 19 ou 20, **caractérisée en ce que** la lancette présente un élément de couplage permettant d'accoupler la lancette à une unité d'entraînement d'un système de prélèvement sanguin.

22. Lancette selon la revendication 21, **caractérisée en ce que** l'élément de couplage permet un accouplement de la lancette selon différentes positions par rapport à l'unité d'entraînement.

23. Lancette selon la revendication 21, **caractérisée en ce que** la lancette présente plusieurs éléments de couplage.

24. Lancette selon la revendication 21, **caractérisée en ce que** les pointes de lancette présentent des orientations différentes sur le corps de lancette.
